# EUROPÄISCHE PATENTSCHRIFT

(11) **EP 2 022 515 B1**
(45) Veröffentlichungstag und Bekanntmachung des Hinweises auf die Patenterteilung: **21.08.2013**
(21) Anmeldenummer: 08168743.6
(22) Anmeldetag: 08.05.2007
(51) Int. Cl.: A61M 1/06

(54) **Milchpumpe**
Milk pump
Pompe à lait

(43) Veröffentlichungstag der Anmeldung: 11.02.2009
(62) Teilanmeldung aus: 07107683.0
(73) Patentinhaber: Trimed AG, 9495 Triesen (LI)
(72) Erfinder: Nüesch, Hansueli, 5453, Remetschwil (CH)
(74) Vertreter: PATWIL AG

(56) Entgegenhaltungen:
- WO-A-01/34226
- WO-A-03/013628
- US-B2- 6 749 582

## Beschreibung

Die Erfindung bezieht sich auf eine Milchpumpe nach dem Oberbegriff des Anspruches 1.

Für gewöhnlich haben in solchen Milchpumpen die Handpumpeneinheit mit der manuellen Betätigungseinrichtung zum Brustansatzstück ein vorbestimmtes, starres räumliches Verhältnis. Nun ist es aber anatomisch so, dass die Stellung der Hand der Frau beim Abpumpen von Milch aus der linken und der rechten Brust an sich eine andere sein sollte, um eine optimal ergonomische Bewegung zu ermöglichen. Um dieses Bedürfnis zufriedenzustellen, ist bereits ein Gerät auf den Markt gekommen, welches, ganz ähnlich, wie dies auch die US-A-6,749,582 später vorgeschlagen hat, ein Verschwenken der Betätigungseinrichtung gegenüber dem Brustansatzstück zu ermöglichen.

Es ist Aufgabe der vorliegenden Erfindung, eine Milchpumpe der eingangs genannten Art so auszubilden, dass die Funktion der Pumpe den Wünschen der Benntzerin angepasst werden kann. Dies gelingt durch die kennzeichnenden Merkmale des Anspruches 1.

Weitere Einzelheiten der Erfindung ergeben sich an Hand der nachfolgenden Beschreibung von in der Zeichnung schematisch dargestellten Ausführungsbeispielen. Es zeigen:
- Fig. 1: einen Vertikalschnitt durch ein erstes Ausführungsbeispiel einer erfindungsge- mässen Milchpumpe etwa nach der Linie I-I der Fig. 5;
- Fig. 2: eine perspektivische explodierte Darstellung der Milchpumpe nach Fig. 1, zu der die
- Fig. 3 und 4: jeweils modular einsetzbare Einheiten für eine fixe Relativlage von Brustan- einsatzstück und Betätigungshebel und für eine verstellbare Relativlage veran- schaulichen;
- Fig. 5: eine hintere Ansicht entsprechend dem Pfeil V der Fig. 2;
- Fig. 6: eine der Fig. 2 ähnliche explodierte Darstellung einer anderen Ausführung oder der Pumpe nach Fig. 1 aber mit anderer Pumpeneinheit; und
- Fig. 7: einen der Fig. 1 ähnlichen Schnitt durch die Ausführung nach Fig. 1, jedoch ver- setzt entlang der Linie VII-VII der Fig. 5.

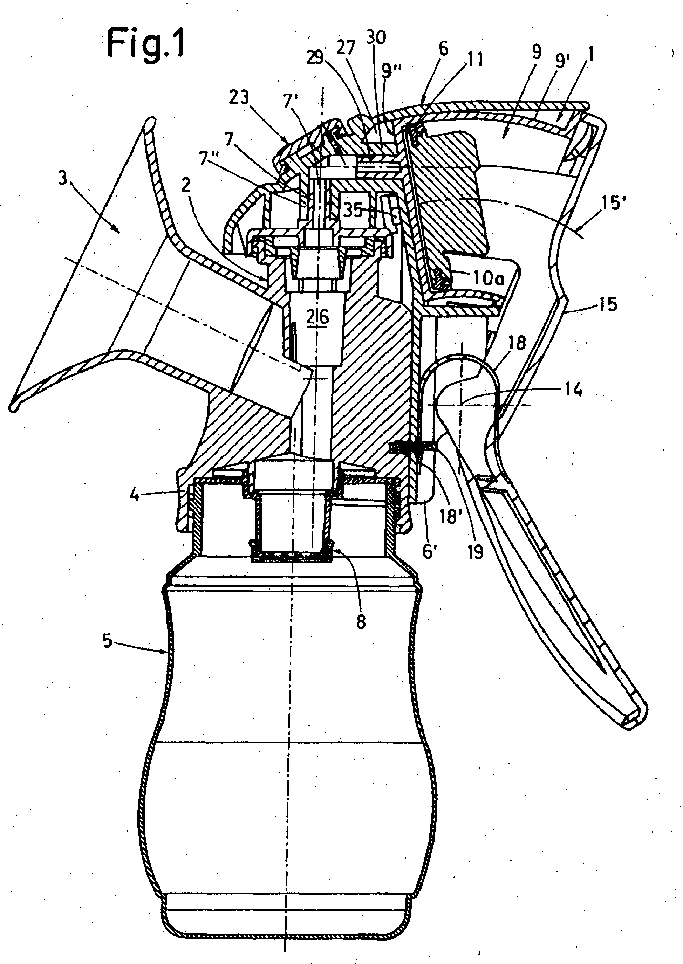

Die in Fig. 1 dargestellte Milchpumpe weist einen oberen Träger 6 mit einer Handpumpeneinheit 1 auf, der auf einen Verbindungsteil 2 aufgesteckt ist. Dazu besitzt der Verbindungsteil 2 einen nach oben ragenden Rohrstutzen 7 mit einer Verbindungsöffnung 7', auf welchen Stutzen 7 ein Rohrstutzen 7" des Trägers aufgesteckt ist. Die beiden Rohrstutzen 7, 7" sind leicht konisch, so dass eine dichte und feste Verbindung entsteht. Der Träger 6 könnte an sich auf die verschiedenste Art ausgebildet sein und beispielsweise aus einzelnen Holmen bestehen; bevorzugt aber ist er, wie dargestellt derart kappenartig ausgebildet, dass er in seinem Inneren die Handpumpeneinheit 1 geschützt aufnimmt.

Der Verbindungsteil 2 umfasst einerseits ein Brustansatzstück 3 oder Horn, anderseits einen Schraubteil 4 mit dem die Pumpe auf einen Milchsammelbehälter 5 aufschraubbar ist. Zwischen Milchsammelbehälter 5 und dem Verbindungsteil 2 liegt in bekannter Weise ein Rückschlagventil 8. Dieses Rückschlagventil 8 ist vorzugsweise so ausgebildet, wie dies in der in der US-A-6,270,474 beschrieben ist, deren Inhalt hier durch Bezugnahme als geoffenbart gelten soll.

An der Oberseite des Trägers 6 mag ein bekannter Reglerknopf 23 angebracht sein, der die Zufuhr von Falschluft regelt und damit auch die Mühe bei der Pumpbetätigung. Eine besonders bevorzugte Ausführung ist beispielsweise in den US-A-6,042,560 und 6,290,671 beschrieben.

Um die Reinigung der nachstehend beschriebenen Handpumpeneinheit 1 nicht durch den Zutritt von Muttermilch noch zu erschweren, ist der Öffnung 7' ein Überlaufventil 26 vorgeordnet. Dieses Überlaufventil ist vorzugsweise so ausgebildet, wie es im EP-Patent Nr. 0 121 825 beschrieben ist.

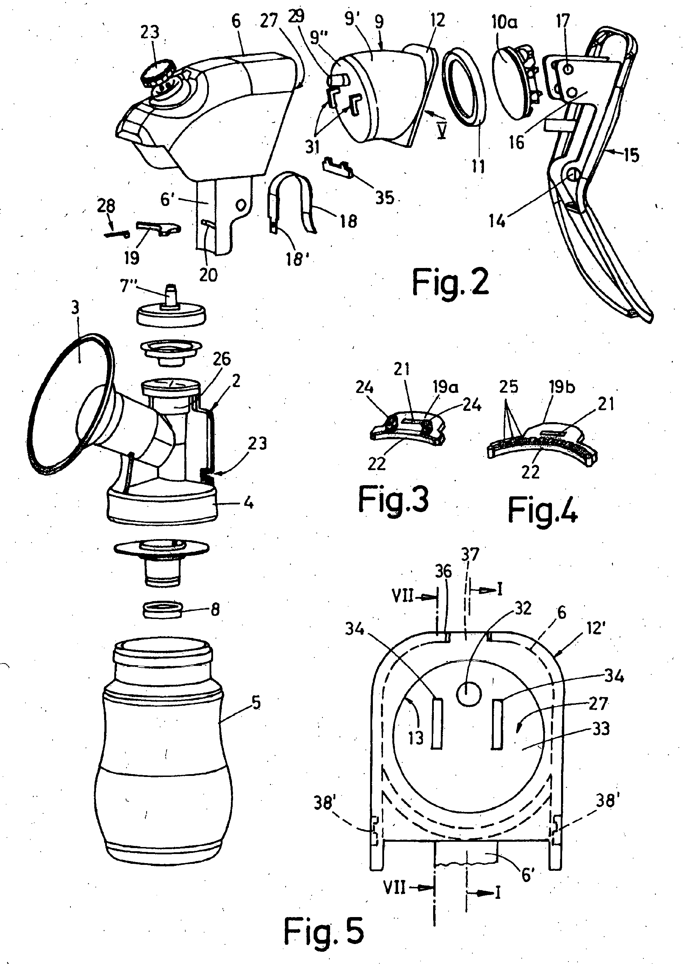

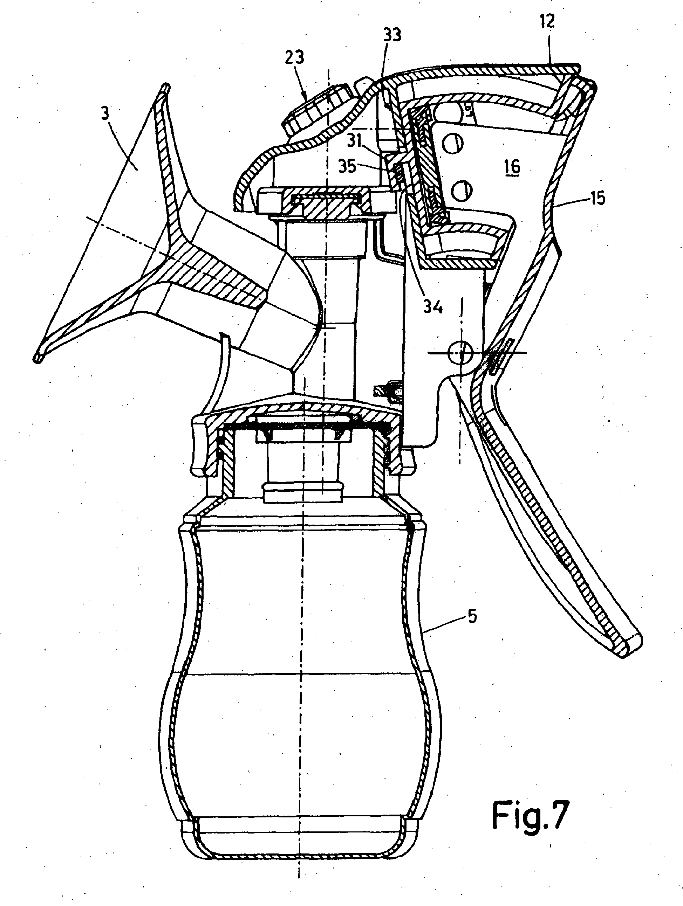

Innerhalb des kappenartigen Trägers 6 ist ein die Pumpeneinheit 1 mit einem Hubraum 9 aufnehmender Hohlraum 27 vorgesehen, wobei der Hubraum 9 von einer Umfangswand 9' umgeben und von einer Stirnwand 9" gegen die Öffnung 7' hin abgeschlossen ist. Dieser Hubraum 9 ist, wie besonders Fig. 2 zeigt, in einem kappenartig ausgebildeten Träger 6 mit im wesentlichen viereckigen Querschnitt mit abgerundeten Ecken (vgl. auch Fig. 5) geschützt eingesteckt und mit diesem lösbar verbunden. Da in dem Hubraum 9 ein Kolben 10a als Saugorgan mittels eines um einen Schwenkpunkt 14 schwenkbaren Betätigungsorganes bzw. (zweiarmigen) Hebels 15 hin- und herbewegt werden soll, muss bzw. soll der Bewegungsbogen 15' möglichst mit der Krümmung der Umfangswand 9' bzw. mit dem gekrümmten Verlauf des Hubraumes 9 übereinstimmen, weil sich andernfalls eine die pumpende Hand ermüdende Reibung ergibt. Um diese Übereinstimmung zu sichern, ist gemäss Fig. 2 zweckmässig ein mit dem Viereckquerschnitt des Trägers 6 übereinstimmender Schild 12 entweder in den ebenso ausgebildeten Hohlraum 27 des kappenartigen Trägers 6 einsetzbar oder auf diese aufsteckbar (vgl. Fig. 7). Wie aus Fig. 1 ersichtlich, kann - bei sehr genauer Fertigung - auf ein solches Einsatzstück bzw. einen solchen Schild 12 auch verzichtet werden, obwohl er als Sicherung gegen Verdrehung des Hubraumes 9 bevorzugt ist.

Wie an Hand der als Abschlusselement dienenden Variante 12' des Rahmens oder Schilds in Fig. 5 ersichtlich ist, hat dieses eine Öffnung 13, durch welche ein mit einem von einem Dichtungsring 11 umgebenen Kolben 10a über Stecklöcher 17 verbindbarer Führungsteil 16 des Hebels 15 hindurch bewegbar ist. Dieser Hebel 15 steht beispielsweise unter der Rückholkraft einer Rückholfeder 18, die vorzugsweise als einfache U-förmige Blattfeder ausgebildet ist und mit einem Ende am Hebel 15 angreift (vgl. Fig. 1). Das andere Ende 18' der Feder 18 ist am Träger 6, gegebenenfalls aber auch am Verbindungsteil 2 befestigt. Dies kann mit einem Verbindungselement 19 geschehen, das beispielsweise einfach von einer Schraube gebildet sein mag. Dabei kann das Verbindungselement 19 gleich einem doppelten Zweck dienen, indem es auch den Träger 6 am Verbindungsteil 2 befestigt.

Fig. 2 zeigt eine bevorzugte Ausführung, bei welcher der nach unten ragende Trägerfuss 6' eine, z.B. schlitzartige, Öffnung 20 aufweist, durch die ein bogenförmiges Befestigungselement 19 wahlweise in der Ausführung 19a als Sperrorgan oder in der Ausführung 19b (vgl. Fig. 3, 4) als eine Relativdrehung des Hebels 15 gegenüber dem Behälter 5 und dem Träger 6 begrenzenden und damit teilweise zulassenden Begrenzungsorgan hindurchsteckbar ist. Dieses bogenförmige Befestigungselement 19a bzw. 19b besitzt jeweils einen Schlitz 21, durch welchen - nach dem Einschieben in den Schlitz 20 - hinter diesem Schlitz 20 das Ende 18' der Feder 18 eingeschoben werden kann und so das Verbindungselement 19 verriegelt. Der vordere Bogen 22 des jeweiligen Befestigungselementes 19 sitzt in einer Schlitzführung 23 (Fig. 2) des Verbindungsteiles 2, wobei im Falle des Befestigungselementes 19a Vorsprünge 24 die Endbegrenzungen des Schlitzes umfassen und so eine fixe Verbindung schaffen, welche keinerlei Relativdrehung von Verbindungsteil 2 und Träger zulässt.

Alternativ aber kann das Befestigungselement bzw. Begrenzungsorgan 19b in den Schlitz 20 eingeschoben und mittels des Endes 18' befestigt werden. Dieses Befestigungselement 19b ist breiter und besitzt Rasten 25, die eine begrenzte Verdrehbarkeit des Trägers 6 gegenüber dem Verbindungsteil 2 gestatten und die jeweilige Relativstellung verrasten. Dadurch ist eine Anpassung der Relativlage beim Pumpen an der linken oder rechten Brust möglich. Durch die Austauschbarkeit der Verbindungselemente 19a bzw. 19b ist es möglich, die Funktion der Pumpe den Wünschen der Benutzerin anzupassen bzw. können so auch leicht verschiedene Modelle der gezeigten Handpumpe erstellt werden, ohne die nötigen Formwerkzeuge zu stark zu verändern. Es versteht sich, dass diese Austauschbarkeit auch unabhängig davon denkbar ist, ob auch der Hubraum 9 oder mindestens eine Wand davon austauschbar ist oder nicht, so dass es sich hier an sich um eine gesonderte Erfindung handelt.

Es versteht sich, dass die beiden Teile 19a, 19b auf die verschiedenste Weise ausgebildet und entweder am Träger 6 bzw. seinem Fuss 6' - um mit den Begrenzungsflächen bzw. einer Gegenrast (vgl. Blattfeder 28 mit Gegenrastnoppe in Fig. 1) am Verbindungsteil 2 zusammenzuwirken - oder am Verbindungsteil 2 vorgesehen werden können - um mit Begrenzungsflächen bzw. einer Gegenrast am Trägerfuss 6' zusammenzuwirken. Auch können die Rasten statt an der Oberseite etwa an der dem Verbindungsteil (oder dem Träger) gegenüberliegenden Stirnseite des Teiles 19b ausgebildet sein. Letztlich liesse sich gegebenenfalls aber auch auf Rasten 25 verzichten und nur eben weiter voneinander beabstandete Vorsprünge (entsprechend den Vorsprüngen 24) vorsehen, was aber weniger bevorzugt ist.

Bezüglich der eben erwähnten Austauschbarkeit des Hubraumes 9 oder mindestens einer Wand 9' und/oder 9" davon sei nun folgendes erläutert.

Wie aus den Fig. 2 und 1 (oder besser 7) hervorgeht, ragt von der Stirnwand 9" des Hubraumes 9 einerseits ein Verbindungsrohrstutzen 29 vor. Gemäss Fig. 1 ist dieser Rohrstutzen 29 in einen Gegenstutzen 30 am Träger 6 steckbar. Da beide Stutzen wieder etwas konisch sind, ergibt sich eine abgedichtete Steckverbindung, welche Leckagen verhindert.

Von der Stirnwand 9" (Fig. 2) ragen aber auch zwei Teile einer Verriegelungseinrichtung, die im dargestellten Ausführungsbeispiel von zwei Haken 31 gebildet sind. Beim Einschieben des Hubraumes 9 in den Hohlraum 27 des Trägers 6 tritt einerseits der Rohrstutzen 29 durch eine kreisrunde Öffnung 32 einer Aufnahmeplatte 33 (Fig. 5, 7) des Trägers 6, anderseits durchsetzen die Haken 31 je einen Schlitz 34 der Platte 33. Nun kann ein Riegel 35, z.B. ein die Stirnwand 9" gegen die Platte 33 pressender Keilriegel, zwischen die Haken 31 geschoben werden, wie besonders aus Fig. 7 ersichtlich ist. Es versteht sich aber, dass dies nur eines von vielen möglichen Beispielen für eine Verrieglung ist, wobei der Fachmann für den Bau von Verrieglungen zahlreiche Abwandlungen kennt. Beispielsweise könnte die Stirnwand 9" seitliche Fortsätze haben, an denen beispielsweise ein Verriegelungsexzenter anzugreifen vermag, um diese Wand 9" gegen die Platte 33 zu pressen. In jedem Falle aber genügt ein Riegel zweierlei Bedingungen:
- er ist leicht und rasch einsetzbar und auch wieder entfernbar, und
- er widersteht grossen Kräften in Bewegungsrichtung des Saugorganes bzw. im konkreten Fall entlang des Bewegungsbogens 15'.

Es ist klar, dass der Ort der Verriegelung, wie oben bereits angedeutet - verschieden gewählt werden kann. Wird er im Bereich der Umfangswand 9' gewählt, dann geht aber in der Regel Platz für den Hubraum verloren. Wird der Ort der Verriegelung am (in der Zeichnung) rechten, offenen Ende des Trägers 6 gewählt, dann wäre der dichte Anschluss zur Öffnung 7' nur erschwert gesichert. Deshalb ist es bevorzugt, wenn die verriegelbare Verbindung 31, 35 an der gegen die zum Behälter 5 führenden Öffnung 7' gewandten Seite angeordnet ist, wie dies aus der Zeichnung zu ersehen ist. Es versteht sich ferner, dass es an sich auch denkbar wäre, die Wand 33 als Stirnwand des Hubraumes 9 auszubilden und nur dessen Umfangswand 9' mit dieser so gebildeten Stirnwand zu verriegeln. In diesem Falle kann sich aber die Abdichtung erschweren bzw. wäre dann sich eine etwa kreisringförmige Dichtung an der Stirnseite dieser Umfangswand 9' nötig, wobei die Reinigung u.U. erschwert sein mag.

Es ist aus der EP 1 231 955 bekannt, den Träger kappenartig auszubilden, allerdings zu einem anderen Zweck, denn dort bildet er gleichzeitig die Umfangsbegrenzung des Hubraumes. Dies hat - ausser der erschwerten Reinigung des Hubraumes - zur Folge, dass man bei der Herstellung stets einen Kompromiss eingehen muss, denn entweder besteht die Wand des Trägers aus einem festen Trägermaterial, welches aber zu mehr Reibung des Kolbens und zu einer erschwerten Betätigung führt, oder man verwendet ein gleitfreundliches Material, das aber dann weniger Formstabilität besitzt. Durch die erfindungsgemäss vorgesehene Trennung der beiden Aufgaben kann nun für beide Anforderungen ein Optimum erzielen, kurz gesagt, indem die Umfangswand des Hubraumes aus einem gegenüber dem Träger differierenden Material gebildet ist. Dabei wird man zweckmässig so vorgehen, dass das Material des Hubraumes einen geringeren Reibungskoeffizienten besitzt als das Material des Trägers und/oder das Material des Trägers eine grössere Festigkeit bzw. Härte als das Material des Hubraumes aufweist. In der Praxis wird man vorteilhaft den Hubraum aus Polyäthylen (PE) oder Polyamid (PA) fertigen und/oder den Träger aus Polycarbonat (PC) oder Polypropylen (PP) herstellen.

Was die schon erwähnte Fig. 5 angeht, so ist aus dieser auch die Befestigung des Schildes 12 am äusseren Ende seines Hohlraumes 27 ersichtlich. Dazu wird er mittels einer, vorzugsweise elastischen, Umfangsspange 12' (Fig. 5, 7) an der Trägerkappe befestigt, d.h. die Spange wird über den Rand des kappenartigen Trägers 6' geschoben und hält bei der Ausführung nach Fig. 1 den Schild 12, bei der Ausführung nach Fig. 7 einen Rand 38 einer topfförmigen Membrane 39, auf die noch eingegangen wird. Jedenfalls kann durch die von einer Kreisform abweichende Form des Hohlraumes 27 (vgl. auch Fig. 5) für eine Verdrehsicherung gesorgt werden, weil dann der gekrümmte Hubraum 9 nur so eingesetzt werden kann, dass seine Krümmung mit der Bogenbewegung 15' (Fig. 1) übereinstimmt.

Die Spange 12' besitzt an ihrer Oberseite eine Schlitzöffnung 36, durch welche ein auch aus der Trägerausführung nach Fig. 7 ersichtlicher Lappen oder Vorsprung 37 eingreift. Wenn also diese Spange 12 aus einem leicht elastischen Material geformt ist, so kann sie ohne weiteres über den Lappen 37 gezogen werden. Die unteren Ränder der Spange 12' können durch Rastnoppen 38' festgehalten werden.

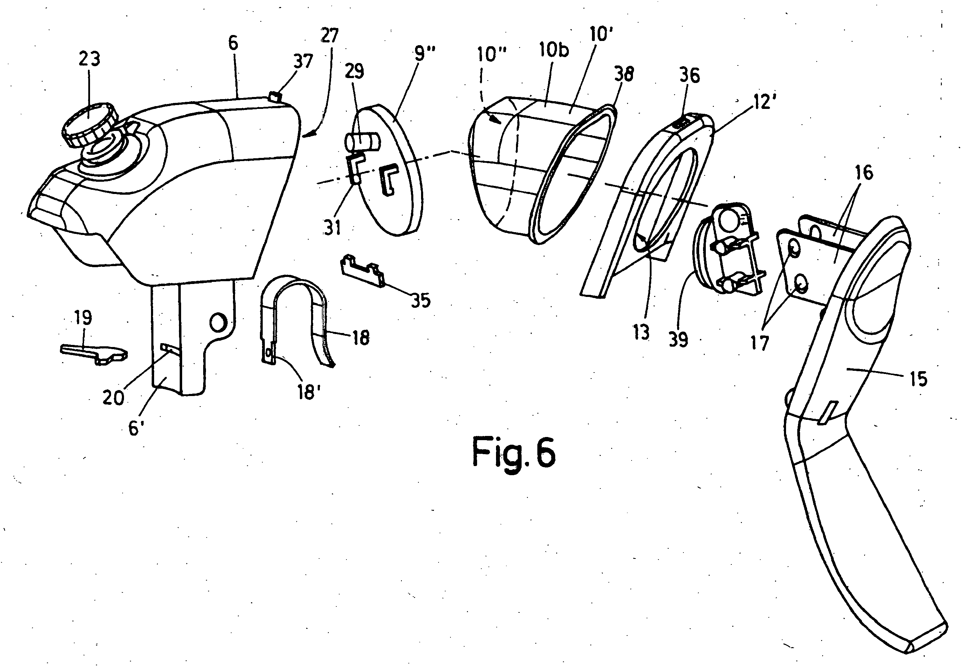

An Hand der Fig. 6 soll gezeigt werden, dass die Erfindung die Herstellung unterschiedlicher Modelle mit geringem Aufwand erlaubt. Für gleiche Teile werden dabei dieselben Bezugszeichen wie in den vorigen Figuren verwendet.

Die Teile an der linken und rechten Seite der Fig. 6 stimmen mit den in Fig. 2 an diesen Seiten gezeigten Teilen überein. Unterschiedlich ist, dass an Stelle des Kolbens 10a (Fig. 2) eine etwa topfförmige Membrane 10b mit einer Umfangswand 10' und einer vorderen, strichliert angedeuteten Stirnwand 10" vorgesehen ist, vorzugsweise so, wie dies in der Europäischen Patentanmeldung EP 071013.46.0 beschrieben ist. An dieser Stirnwand ist ein die kreisrunde Öffnung 13 (vgl. Fig. 5) durchgreifender Anschlussteil 39 befestigt, der seinerseits mit dem Führungsteil 16 des Hebels 15 über die Stecklöcher 17 mittels Steckstiften verbunden ist. Nun könnte an sich auch für die Topfmembrane 10b ein gekrümmter Zylinderraum entsprechend dem Hubraum 9 vorgesehen werden. Es wurde aber oben bereits auf den besonderen Vorteil der lösbaren Anbringung des Hubraumes 9 hingewiesen, der u.a. in der Möglichkeit der Anpassung des Kunststoffes an die verschiedenen Reibungsverhältnisse liegt. Bei Verwendung einer Membrane 10b braucht ja aber auf den Reibungskoeffizienten des Materials gar keine Rücksicht genommen werden, und deshalb genügt es, wenn an Stelle des ganzen Hubraumes 9 gemäss Fig. 2 nur die Stirnwand 9" zur Abdeckung der Schlitze 34 bzw. für die Verbindung der Rohrstutzen 29, 30 (vgl. Fig. 1, 5, 6) eingesetzt wird. Dagegen steht der gesamte Hohlraum 27 des Trägers 6 zur Unterbringung der Topfmembrane 10b zur Verfügung, d.h. sie kann im Querschnitt grösser als der Kolben 10a sein und daher auch eine grössere Pumpleistung erbringen. Zur Befestigung der Membrane 10b am Träger 6 ist die bereits an Hand der Fig. 5 beschriebene, vorzugsweise elastische Spange 12' vorgesehen, welche den Rand 38 der Membrane 10b am Rande des Trägers 6 und dessen Hohlraumes 27 festklemmt.

## Patentansprüche

1. Milchpumpe welche folgendes umfasst:
a) eine Handpumpeneinheit (1) mit einem im wesentlichen von einer Umfangswand (9') und einer stirnseitigen Abschlusswand (9") umschlossenen Hubraum (9) und ein Saugorgan (10a, 10b);
b) einen Träger (6) für diese Handpumpeneinheit (1), die oberhalb einer zu einem Behälter (5) führenden Öffnung (7') des Trägers (6) angeordnet ist und deren Hubraum (9) lösbar an den Träger (6) angeschlossen ist ;
c) einen Verbindungsteil (2), welcher ein Brustansatzstück (3) umfasst, das mit dem Träger (6) verbunden ist;
d) eine manuelle Betätigungseinrichtung (15), wie einen Hebel (15) - vorzugsweise einen zweiarmigen, in einem Mittelbereich schwenkbar gelagerten Hebel (15) - mit dem das Saugorgan (10a, 10b) der Handpumpeneinheit (1) im Hubraum (9) hin- und herbewegbar ist;
**dadurch gekennzeichnet, dass** der Verbindungsteil (2) mit einer Verbindung zu der zum Behälter (5) führenden Öffnung (7') versehen ist, und dass dieser Verbindungsteil (2) und/oder der an ihm befestigbare Träger (6) ein ein Lösen erlaubendes, modular einsetzbares Befestigungselement (19) - alternativ in der Ausführungsform für ein gegen eine Drehung der manuellen Betätigungseiririchtung (15) um die Achse der zum Behälter (5) führenden Öffnung (7') sicherndes, lösbares Sperrorgan (19a), oder für ein eine solche Drehung begrenzenden und damit teilweise zulassenden Begrenzungsor an (19b) - aufweist.

2. Milchpumpe nach Anspruch 1, **dadurch gekennzeichnet, dass** das modulare Befestigungselement bogenförmig ist und dass der Träger (6) einen nach unten ragenden Trägerfuss (6') mit einer, z.B. schlitzartigen, Öffnung (20) aufweist, durch die das, insbesondere bogenförmige, modulare Befestigungselement (19) hindurchsteckbar ist.

3. Milchpumpe nach Anspruch 1 oder 2, **dadurch gekennzeichnet, dass** der Hebel (15) von einer als U-förmige Blattfeder ausgebildeten Rückholfeder (18) belastet ist, von der ein Endbereich in einen Schlitz (21) des Sperr- oder Begrenzungsorganes (19a bzw. 19b) zum Verriegeln desselben einschiebbar ist.

4. Milchpumpe nach einem der vorhergehenden Ansprüche, **dadurch gekennzeichnet, dass** das die Drehung begrenzende und damit teilweise zulassende Begrenzungsorgan (19b) Rasten (25) besitzt, die die jeweilige Relativstellung zwischen manueller Betätigungseinrichtung (15) und Träger (6) verrasten.

## Claims

1. Milk pump which comprises:
a) a manual pumping unit (1) including a displacement space (9) substantially enclosed by a circumferential wall (9') and a frontal closing wall (9") as well as a suction member (10,a, 10b);
b) carrier means (6) for this manual pumping unit (1) arranged above an opening (7') of the carrier means (6) which leads to a receptacle (5), the displacement space (9) being releasably connected to the carrier (6)
c) a breast shield (3) which is connected to the carrier (6) via a connection portion (2);
d) manual actuation means (15), such as a lever (15), preferably a tow-armed lever (15) pivoted in a mean range, by which the suction member (10a, 10b) of the manual pumping unit (1) may be moved to and fro within the displacement space (9);
**characterised in that** the connection portion (2) is provided with a connection to the opening (7') which leads to the receptacle (5), and that this connection portion (2) and/or the carrier (6) to be fastened to it includes a fastening element (19) which allows releasing and is insertable in a modular manner, alternatively as an embodiment of a releasable locking element (19a), which secures against rotation of the manual actuation means (15) about the axis of the opening (7') leading to the receptacle (5) or as a limitation element (19b) which delimits such rotation and, thus, partially allows it.

2. Milk pump according to claim 1, that the modular fastening element is arcuate, and that the carrier (6) comprises a carrier foot (6'), which projects downwardly and has a, e.g. slot-shaped, opening (20), through which the, particularly arcuate, modular fastening element (19) may be plugged through.

3. Milk pump according to claim 1 or 2 **characterised in that** the lever (15) is charged by a return spring (18) formed as a U-shaped leaf spring, a terminal portion of which being able to be inserted into a slot (21) of the locking or limitation element (19).

4. Milk pump according any of the preceding claims, **characterised in that** the limiting element (19b), which delimits rotation and, thus, partially allows it, includes notches (25), which catch the respective relative position between the manual actuation means (15) and the carrier (6).

## Revendications

1. Pompe à lait qui comprend :
a) une unité de pompe manuelle (1), qui inclut une cylindrée (9) sensiblement entourée par une parois périphérique (9') et une parois de fermeture frontale (9"), et un élément aspirateur (10a, 10b) ;
b) un porteur (6) pour cette unité de pompe manuelle (1), qui est disposée au-dessus d'une ouverture (7') du porteur (6), menant à un réceptacle (5), dont le cylindrée (9) est relié au porteur (6) d'une manière détachable ;
c) une pièce d'assemblage (2), comprenant une pièce d'application contre la poitrine (3) reliée au porteur (6) ;
d) un dispositif d'actuation manuelle (15), comme un levier (15), de préférence un palonnier, étant pivotant dans une zone centrale, par lequel l'élément aspirateur (10a, 10b) de l'unité de pompe manuelle (1) peut être mû dans le deux sens dans la cylindrée (9) ;
**caractérisé en ce, que** la pièce d'assemblage (2) est pourvue d'une connexion à l'ouverture (7') menant au réceptacle (5), et que cette pièce d'assemblage et/ou le porteur (6), capable à être fixé à elle, comprend un élément de fixage (19) permettant un détachement et étant utilisable d'une manière modulaire, alternativement dans une forme de construction pour un organe de verrouillage (19a) dégageable, qui fiabilise contre une rotation du dispositif d'actuation manuelle (15) autour de l'axe de l'ouverture menant au réceptacle (5), ou pour un organe de délimitation (19b), qui délimite une telle rotation, et ainsi l'admet partiellement.

2. Pompe à lait selon la revendication 1, **caractérisé en ce, que** l'élément de fixage (19) modulaire est arqué, et que le porteur (6) comprend un pied de porteur (6') qui fait saillie ver le bas, et qui comprend une ouverture (20), par exemple d'un type de fend, à travers de laquelle l'élément de fixage (19) modulaire, en particulier en forme d'arque, peut être enfiché.

3. Pompe à lait selon la revendication 1 ou 2, **caractérisé en ce, que** le levier (15) est chargé d'un ressort de rappel formé comme un ressort à lames (18) en forme d'un U, dont une zone terminale peut être introduit dans une fente (21) de l'organe de verrouillage ou de délimitation (10a ou 19b) pour le verrouiller.

4. Pompe à lait selon une quelconque des revendications précédentes, **caractérisé en ce que** l'organe de délimitation (19b), qui délimite la rotation, et ainsi l'admet partiellement, possède des encoches (25) pour enclencher la position relative respective entre le dispositif d'actuation manuelle (15) et le porteur (6).
